Europäisches Patentamt

European Patent Office

Office européen des brevets

(11) Publication number: **0 295 826**
**A1**

## EUROPEAN PATENT APPLICATION

(21) Application number: 88305273.0

(51) Int. Cl.⁴: **A61K 37/50**

(22) Date of filing: 09.06.88

(30) Priority: 09.06.87 US 59806

(43) Date of publication of application:
**21.12.88 Bulletin 88/51**

(84) Designated Contracting States:
**AT BE CH DE ES FR GB GR IT LI LU NL SE**

(71) Applicant: **INTERNATIONAL CARDIOVASCULAR MEDICINE, INC.**
**18345 Ventura Boulevard Suite 211**
**Tarzana California 91356(US)**

(72) Inventor: **Burman,Michael Herman M.D.**
**23472 Rolling View Drive**
**Hilden Hills California 91302(US)**

(74) Representative: **Nash, Keith Wilfrid et al**
**KEITH W. NASH & Co. Pearl Assurance**
**House 90-92 Regent Street**
**Cambridge CB2 1DP(GB)**

(54) Method of using superoxide dismutase during surgical procedures.

(57) A method is disclosed for treating global ischemia resulting in free radical formation, for example, during open heart surgery by utilizing an anti-free radical effective amount of superoxide dismutase. During open heart surgery, the use of superoxide dismutase protects the heart from structural damage during re-oxygenation.

EP 0 295 826 A1

Xerox Copy Centre

## METHOD OF USING SUPEROXIDE DISMUTASE DURING SURGICAL PROCEDURES

## BACKGROUND OF THE INVENTION

### Field of the Invention

This application for United States Letters patent is a continuation-in-part of U.S. Serial No. 059,806, filed June 9, 1987.

This invention relates to a method for performing surgical procedures by utilizing an effective amount of superoxide dismutase to protect the organ from structural damage during re-oxygenation.

### DESCRIPTION OF THE PRIOR ART

A copper containing protein was isolated from erythrocytes and found to be one of a family of water-soluble metalloprotein congeners which was assigned the non-proprietary name "orgotein" by the U.S. Adopted Name Council. It was determined that these congeners possessed enzymatic activity which dismuted superoxide radicals to peroxide and molecular oxygen. Thus, the name, superoxide dismutase (SOD) was adopted.

It has been theorized that superoxide dismutase may perform, among others, the role of protecting cells from the toxic effects of physiologically produced superoxide radicals, as well as against the deleterious effects of ultraviolet radiation.

Superoxide dismutases which contain copper and zinc in their active sites are found in the cytoplasm of eukaryotes. It has been found that these enzymes are dimeric molecules which exhibit a very high degree of homology in their amino acid sequence (C. Petersen et al., Carlsberg Res. Commun. Vol. 42, p. 391-395, 1977) and have related physico-chemical properties (A.E.G. Cass et al, Carlsberg Res. Commun. Vol. 43, p. 439-449, 1978). Another class of superoxide dismutases which contain iron or manganese in their active sites are found in prokaryotes and in eukaryotic mitochondria. In this class there is also a high similarity in amino acid composition and N-terminal amino acid sequence. However, there is no significant homology between the two classes of superoxide dismutases.

It is apparent that the function of the superoxide dismutases is to protect the cells in aerobic organisms against the toxic effects of the superoxide radical, which is a by-product of the reaction of oxygen in the organism. It is believed that the superoxide radical is involved in various inflammatory processes in tissues and that it may contribute in the pathogenesis of rheumatoid arthritis. It has therefore been proposed to use superoxide dismutase for the general treatment of inflammations, and perhaps rheumatoid arthritis. The therapeutic effect of Cu,Zn-superoxide dismutase on inflammatory diseases has been confirmed by experiments. It would accordingly be of great importance if it were possible to provide a process for recovering Cu,Zn-superoxide dismutase on an industrial scale in a high yield. This has now been achieved by a number of prior art processes.

Superoxide dismutase is widely distributed in animal and plant organisms. The enzyme functions to eliminate active oxygen from those organisms where active oxygen is generated in the course of biochemical reactions and wherein active oxygen acts as a cytotoxin.

Superoxide dismutase obtained in its pure state from the red blood cells of higher animals is a protein metal chelate, which is named "orgotein", as discussed hereinbefore.

A number of references teach various uses and extraction sequences for superoxide dismutase.

U.S. Patent No. 3,579,495 describes a method for isolating orgotein from red blood cells. The method disclosed therein involves first removing hemoglobin from the listed red blood cells by precipitating the hemoglobin with an organic solvent at low temperature and near neutral pH. The organic solvent is a water-immiscible solvent such as methylene chloride and chloroform, and a small amount of a water-miscible solvent can be added to increase the contact between the hemoglobin and the precipitating solvent. After precipitation of the hemoglobin, the hemoglobin is removed. The hemoglobin-free supernatant is then heated in the presence of a divalent metal cation such as $Mg^{++}$, $Cu^{++}$, or $Mn^{++}$, to remove proteinaceous impurities. The divalent metal cations are then removed and the isolated orgotein recovered from the supernatant.

U.S. Patent No. 3,920,521 discloses use of superoxide dismutase for inhibiting the degradation of auto-oxidizable substances, notably for preserving foodstuffs, bacteria, viruses, and related oxidizable substances.

U.S. Patent No. 4,029,819 discloses a method for the production of superoxide dismutase which is extracted from marine bacterial strains and characterized in that it is primarily non-hematinic iron or copper and zinc.

U.S. Patent No. 4,129,644 discloses that superoxide dismutase may be used as a component of a hygienic or cosmetic composition.

U.S. Patent No. 4,435,506 discloses that superoxide dismutase may be isolated by contacting red blood cells containing proteinaceous impurities with a water-miscible organic solvent in a given pH and temperature range, removing the impurities and obtaining purified superoxide dismutase.

U.S. Patent No. 4,346,174 discloses a process for isolating superoxide dismutase from red blood cells by heating hemolyzed red blood cells in the presence of at least one monovalent inorganic neutral salt and at least one transition metal salt, eliminating precipitate from the hemolyzed red blood cells by filtration or centrifugation to obtain a solution, adding further at least one transition metal salt at least once to the solution under application of heat, and eliminating the precipitate from the solution.

U.S. Patent No. 4,340,675 discloses that copper-zinc-superoxide dismutase may be recovered from yeast by plasmolysis using a small amount of ether or other water immiscible organic solvent and subsequent autolysis in water following which the precipitate is removed and the superoxide dismutase is purified and isolated from the residual liquid, by chromatography on carboxylmethyl cellulose.

U.S. Patent No. 4,388,406 discloses a process for isolating copper-zinc-superoxide dismutase from aqueous solutions containing the enzyme together with accompanying proteins by chromatography of the solution at a pH of 4.7 to 5.5 on a cation exchange resin such as carboxymethyl celluloses, cross-linked dextrans substituted with carboxymethyl groups, sulfopropyl groups or cross-linked agaroses substituted with carboxymethyl groups.

U.S. Patent No. 4,563,349 discloses a new superoxide dismutase belonging to the genus Serratia which may be immobilized by binding it to a water-soluble carrier to produce a product having pharmacological effects such as decreased antigenicity and increased anti-inflammatory activities. The Serratia SOD enzyme is disclosed to have a molecular weight of about $4.8 \times 10^4$.

It is known that superoxide dismutase exists in human blood. However, the amount of superoxide dismutase is so small that its effectiveness is negligible in preventing structural damage of the heart during re-oxygenation following surgery.

## DETAILED DESCRIPTION OF THE INVENTION

The present invention is directed to a method of treating global ischemia which results in free radical formation followed by reperfusion, in a mammal by administering an anti-free radical effective amount of superoxide dismutase.

It has now been found that when safe and effective amounts of superoxide dismutase are applied to the heart during heart surgery, a significant increase in hemodynamic function to near normal occurs during re-oxygenation.

Superoxide dismutase exists in a number of known varieties which may differ in their physiochemical properties. A known superoxide dismutase includes one obtained from Escherichia coli B as described in The Journal of Biological Chemistry Vol. 245, No. 22, p. 6176-6181, 1970 and one derived from Bacillus stearothermolphilus having a molecular weight of 40,000. The superoxide dismutase according to the present invention is different since the above molecular weight figures are significantly different from the molecular weight of the superoxide dismutase of the present invention, which is about $4.8 \times 10^4$.

The superoxide dismutase useful in accordance with the present invention can be derived from animal, bacterial or vegetable origins. Representative superoxide dismutases include, without limitation, those extracted from bacteria, from mushrooms and from blood.

Representative superoxide dismutase extracts of bacterial origin include, preferably (particularly), extracts of Escherichia coli; representative superoxide dismutase extracts of mushroom origin include, for example, extracts of Pleurotus olearius; representative superoxide dismutase extracts from blood include, in particular, erythrocuprein.

Also usefully employed in the present invention are superoxide dismutase extracts of marine bacterial strains, such as for example, strains of Photobacterium phosphoreu, Photobacterium leiognathi or Photobacterium sepia. Representative useful strains include the strains of Photobacterium phosphoreum ATCC No.

EP 0 295 826 A1

11040, Photobacterium leiognathi ATCC No. 25521, Photobacterium sepia ATCC No. 15709, Escherichia coli ATCC No. 15224 and Pleurotus olearius Gillet (Laboratoire de Cryptogamie de Paris).

The superoxide dismutase used according to the present invention can be prepared by the various methods described in the literature. The measurement of the activity of superoxide dismutase was performed in accordance with McCord and Fridovich's method proposed in the Journal of Biochemistry, Vol. 244, 6049 (1969). The isoelectric point of the superoxide dismutase is near pH 5.5.

Superoxide dismutase appears to be generally non-toxic to living organisms and, immunologically, it is not recognized as a foreign material in living organisms. As a result, the superoxide dismutase of the present invention can be administered parenterally, for example, intravenously.

The superoxide dismutase is preferably added during open heart surgery. It may, however, be administered for example, during pretreatment of the heart by addition to a cardioplegic solution, pretreatment with catalase, as a bolus injection alone or in combination with catalase immediately before aortic cross clamp, or immediately following aortic cross clamp removal, by bolus injection alone or in combination with catalase before and after cross clamp removal with prolonged infusion during the early phases of resumption of normal and spontaneous circulation.

With regard to the type of carriers in which the SOD or SOD/catalase may be dissolved, any physiologically acceptable vehicle may be used. Examples are physiological sodium chloride solution (0.9%), blood, plasma, buffered physiological saline solution, cardioplegic solution, 5 percent dextrose and sterile water. Cardioplegic solution is a "cocktail" of a variety of ingredients which varies geographically according to the proclivities of the individual cardiovascular surgeon. Two types of cardioplegic solutions are used frequently depending upon the point of administration. For induction of cardiac arrest, the potassium chloride content is elevated. Solution A is used which contains 5 percent dextrose, 0.2 percent sodium chloride (250 ml), the buffer THAM (100 ml), the anticoagulant citrate phosphate dextrose solution (so-called CTD) (20 ml), and potassium chloride (40 milliequivalents). Solution B which is used following arrest and maintenance of cardioplegia, is 500 ml of 5 percent dextrose and 0.2 percent sodium chloride, 200 ml THAM, 40 ml CTD, and 30 milliequivalents of potassium chloride. These solutions may include other nutrients thought to support the heart during anoxia by supplying the substrates for anaerobic metabolism. Thus, variations include increased concentrations of glucose as well as the addition of amino acids, magnesium, calcium and physiologically acceptable electrolytes.

The dosing of superoxide dismutase alone or with catalase ranges from about 0.5 to 50 mg/kg body weight and preferably about 1 mg to 20 mg per kg body weight. The dose could be divided, depending upon the method and number of administrations. For example, a bolus injection, representing about 15 to 35 percent of the total dose, can then be followed by the residual dosage as an infusion over an appropriate time period. An average dose would most likely be about 5 milligrams per kilogram of SOD and 5 milligrams per kilogram for catalase.

According to the method of administration, SOD or SOD/catalase would be prepared proximate to the time of surgery, e.g. by addition to a cardioplegic solution or to a separate solution to be used in conjunction with a cardioplegic solution. The concentration of SOD or SOD/catalase in the solution or solutions is determined by the desired method of administration, i.e. whether as a single bolus injection, bolus plus infusion, or complete infusion.

With regard to the surgical procedure, cardiopulmonary bypass proceeds in several principal steps, the first being cannulation. After the chest cage is opened, cannulae (tubes) are positioned at various locations in the arteriovenus circuit, thereby preparing the patient for cardiopulmonary bypass. At the same time, an extracorporeal pump/oxygenator is prepared and primed with appropriate solutions to continue circulation of the blood while the heart is arrested.

Next, the heart and sometimes the patient generally are cooled to decrease bodily functions. One effective means for cooling the heart in particular is utilization of cold isotonic saline solution. While the heart is suspended in a cradle created by the opening of the pericardium and its suturing to the chest wall, thereby suspending the heart in a hammock, the heart is immersed in cold isotonic saline solution. After cooling of the heart takes place, the key step of cross clamping of the aorta which is performed simultaneously with the placing of a vent into the left ventricle for the removal of excess pressure is carried out. This step is either preceded by or quickly followed by perfusion of a potassium-rich solution into the coronary circulation to arrest the heart. Injection of the so-called cardioplegic solution is accomplished by either direct injection into the aortic root proximal to the aortic cross clamp, and in communication with the coronary arteries, or by direct needle insertion into the proximal coronary arteries themselves.

Once this key step is completed, circulation is provided by the extracorporeal pump oxygenator for the duration of the procedure. Throughout this time the heart is kept at hypothermic temperatures by continuous or intermittent bathing with the cold isotonic solution. At the same time infusion of the

4

cardioplegic solution is performed either intermittently or continuously as described hereinabove.

During this time the heart muscle, although hypothermic, is anoxic. At the termination of the portion of the procedure which requires arrest of the heart, such as during aortocoronary bypass grafting, valvular replacement, or the like, upon resumption of circulation and bathing of the heart in hyperoxygenated blood, a true reperfusion state occurs representing the transition from cold anoxia to warm hyperoxia. It is at least prior to or at this time that an infusion of superoxide dismutase is added to protect the heart muscle from the generation of oxygen free radicals. Clearly, the superoxide dismutase may be added as a separate or supplemental treatment, if desired.

Infusion of superoxide dismutase continues until the heart is returned to normal rhythms, support is removed and the operation is concluded.

The following are examples which fully describe the present invention, but are deemed not to be limiting thereof.

## METHODS

Animals: Mixed sex Yucatan mini-pigs (30-40 kg) were used after a 12 hour fast. All pigs received humane care in compliance with the "Principals of Laboratory Animal Care" formulated by the National Society for Medical Research and the "Guide for the Care and Use of Laboratory Animals" prepared by NIH.

Anesthesia: General anesthesia was achieved with ketamine hydrochloride (22 mg/kg IM and 1.1 mg/kg IM). Thereafter, intravenous infusion of sodium pentobarbital was administered to achieve deep anesthesia. Ventilation was controlled with a volume ventilator using 100 percent inspired oxygen and a cuffed endotracheal tube. Arterial $pCO_2$ was maintained at 35 to 45 millimeters of mercury and pH was held between 7.35 and 7.45.

Cardiopulmonary Bypass: After anesthesia, thoracotomy, catheter placement and baseline hemodynamic and metabolic measurements were taken. Sodium heparin (2-3 mg/kg) was administered via the right atrium to assure activated clotting time was greater than 400 seconds. Thereafter, aortic and right atrial cannulae were placed and cardiopulmonary bypass begun. Aortic cross-clamping was used to achieve global myocardial ischemia. Core body temperature was cooled to 25° C, and the left ventricle was vented via the pulmonary vein. Cardioplegia was infused at 0, 20 and 40 minutes and continued for a total of 60 minutes. Electrical defibrillation was applied as necessary.

Cardioplegic Solutions: Standard cardioplegic solutions were made as follows: 500 milliliters of dextrose and 1/4 normal saline is added to 200 milliliters of Tham buffer (7.2 g Tham). Then, 50 milliliters of CPD (citrate, phosphate, dextrose) was added, along with 30-70 milliequivalents of KCl. Each 760 ml of experimental cardioplegic solution also contained either (1) 9.5 mg bovine serum albumin, 19.0 mg lactose and 1.9 mg tris HCl (as a control) or (2) manganese SOD; 10 micrograms per liter; assuming a specific activity of 3000 units per milligram of protein

Control concentrations were determined by the following assumptions:

SOD has a specific activity of 2400 units/mg;

SOD in cardioplegic solution has a concentration of 30,000 units/liter;

SOD per each vial:

5 mg enzyme

10 mg lactose

5 μmoles tris HCl

The entire research team was blinded as to the identity of the cardioplegic solutions containing either SOD or placebo. Thus, in any given experiment, it was unknown whether the control or SOD cardioplegic solution was being used.

Hemodynamic Measurements: Pressure transducer-tipped catheters were placed in the left ventricle and aorta for the measurement of pressures as well as a calculation of maximum positive and negative dP/dT. A balloon-tipped Swan Ganz catheter was placed in the pulmonary artery for the measurement of right atrial, pulmonary artery, and pulmonary capillary wedge pressures as well as cardiac output.

Assessment of Left Ventricular Contractility: A Webster 12 pole volume conductance catheter was placed in the left ventricle of each mini-pig, with a 3 French transducer-tipped catheter within its central lumen. Data was acquired digitally, and individual pressure-volume loops reconstructed using the halcom system. Loops were acquired during a series of preload reductions (by intermittent 10 second inferior vena cava occlusion), and the end-systolic pressure volume relationship and volume intercept were determined.

Coronary Sinus Blood Flow: In order to measure coronary sinus blood flow, a catheter was placed into the coronary sinus and flow rates measured via continuous thermodilution.

Myocardial Metabolism: Three indices of myocardial metabolism were utilized including, myocardial lactate production, myocardial oxygen consumption, and myocardial creatine kinase MB release as measured by column chromatography.

Light and Electron Microscopy: Portions of the left ventricle were fixed by perfusion with cold buffered 1% glutaraldehyde/4% formalin for 20 minutes followed by immersion in cold buffered glutaraldehyde/formalin for 24 hours. The hearts were then cut into four sections perpendicular to the long axis, fixed with osmium tetroxide in 0.1 M phosphate buffer for 90 minutes, dehydrated in a series of graded alcohols and xylene and embedded in epoxy resin. Thick sections (1 micron) were stained with hematoxylin and eosin for light microscopy. Ultrathin sections from the left ventricular free wall (90 Angstroms) were stained with lead and uranyl acetate for electron microscopy.

Transmural needle biopsies of the left ventricular free wall were taken prior to aortic cross clamping and at 60 and 120 minutes post reperfusion. Biopsies were immediately placed in 10% neutral buffered formalin for histological evaluation, and 2 1/2% buffered glutaraldehyde for electron microscopic examination.

A combination of light (paraffin and 1 μm plastic) and electron microscopic sections were examined to determine the extent of any ischemic damage present. Cell structure and ischemic damage were determined by the following parameters:

Normal:
    general appearance of cell structure:
    compact
    free of edema
    abundant glycogen
    mitochondria have normal basophilic matrix
    normal appearance of organelles
    endothelial cells are thin
    sarcolemma T tubules appear normal

Mild damage:
    vacuolization within cells
    mild edema
    mild contracture of sarcolemma near intercalated disc
    loss of mitochondrial structure
    focal blebbing of sarcolemma

Severe damage:
    myofibril disruption with severe contraction band formation
    severe sarcolemmal distortion and blebbing
    extensive cellular and mitochondrial edema
    abnormal mitochondrial ultrastructure
    vacuolization or blebbing of endothelial cells


## EXPERIMENTAL PROTOCOL


A total of 6 pigs were randomly assigned to receive the control and SOD cardioplegic solutions in a blinded study. The SOD group were assigned the numbers 1, 4, and 6. The control group were assigned the numbers 2, 3, and 5. Baseline measurements as described above were obtained after anesthesia induction and stabilization and prior to aortic cross-clamp and global ischemia. Thereafter, these same groups of measurements were determined at 30 minutes of global ischemia, 60 minutes of global ischemia, at time 0 of cardiopulmonary bypass, at 30 and 60 minutes thereof, and during 120 minutes of recovery. The number of DC (direct current) counter shocks required to return the heart to sinus rhythm after termination of aortic cross-clamp was also noted.

### RESULTS

Data analysis indicates that the SOD treated group differed significantly from the control group in the following ways:

SOD treated animals showed a rapid return to normal cardiovascular function following cessation of cardiopulmonary bypass in all cases. There was a 30 percent reduction in the time to recovery of 90 percent of normal left ventricular function as measured by dP/dT, cardiac output, and left ventricular filling pressure. In addition, constructed left ventricular pressure volume loops indicated that left ventricular function returned to the baseline within 15 minutes in SOD treated animals. None of the placebo treated hearts resumed hemodynamic function following the cessation of pulmonary bypass.

TABLE 1

| Baseline | SOD Treated Experiments 1,4,6 | Placebo Treated Experiments 2,3,5 |
|---|---|---|
| Systolic Pressure | 85.3 ± 4.9 mmHg | 99.3 ± 3.7 |
| LVEDP | 11.0 ± 2.3 mmHg | 11.3 ± 3.9 |
| Cardiac Output | 3.96 ± 1.5 L/min | 3.25 ± 0.4 |
| Termination | | |
| Systolic Pressure | 72.0 ± 8.4 mmHg | 0 |
| LVEDP | 9.0 ± 3.6 mmHg | 0 |
| Cardiac Output | 3.2 ± 0.7 L/min | 0 |

LVEDP = Left ventricular end diastolic pressure
L/min = Liters/minute

Light Microscopy: Examination of sections from the left ventricle, examined blindly by cardiac pathologists, indicated that the degree of damage to the left ventricle on a light microscopic basis was significantly less in the SOD treated animals. Pathologists were shown the sections and asked to group according to fixed histologic criteria. This was performed in the blinded manner and repeated three times on successive weeks. There was a 90 percent intraobserver precision. Light microscopic analysis verified that microscopic damage was significantly less in the SOD treated group.

Electron Microscopic Changes: Similar blinded analyses of sections performed by electron microscopy were examined by the cardiac pathologists. Based on ultrastructural criteria, again SOD treated animals showed less damage, including more normal mitochrondrial architecture, reduction in intracellular vacuolization, reduction in calcium accumulation, and maintenance of the integrity of the cell surface as well as contractual protein architecture.

Based on the above, it is clear that supplementation of standard cardioplegic solution by the addition of manganese superoxide dismutase significantly protects swine left ventricular myocardium following global ischemia and 60 minutes of cardiopulmonary bypass.

Morphological data was reviewed after the preliminary six animals (3 control and 3 SOD) had been examined. Additional sections or biopsies may be added, or conversely, a lesser number of sections may be subsequently evaluated depending on severity and distribution of pathological changes.

The present invention is applicable to body situations where there is free radical formation as a result of global ischemia followed by reperfusion as opposed to regional ischemia wherein blood flow prior to reperfusion is always positive. The invention is particularly applicable to open heart surgery in humans.

The embodiments described are intended to be merely exemplary and those skilled in the art will be able to make variations and modifications in them without departing from the spirit and scope of the invention. All such modifications and variations are contemplated as falling within the scope of the following claims, and the invention as disclosed.

**Claims**

1. A method for treating global ischemia resulting in free radical formation in a mammal which comprises administering an anti-free radical effective amount of superoxide dismutase.

2. A method for treating global ischemia of the heart resulting in free radical formation in a mammal which comprises administering an anti-free radical effective amount of superoxide dismutase.

3. The method of claim 1 wherein the superoxide dismutase is administered in combination with catalase.

4. The method of claim 1 wherein said anti-free radical effective amount of superoxide dismutase is from about 0.5 mg to about 50 mg of superoxide dismutase per kilogram body weight.

5. The method of claim 4 wherein said anti-free radical effective amount of superoxide dismutase is from about 1 mg to about 20 mg of superoxide dismutase per kilogram body weight.

6. The method of claim 1 wherein said superoxide dismutase is administered in a cardioplegic solution.

7. The method of claim 1, wherein said superoxide dismutase is administered by bolus injection.

8. The method of claim 1 wherein said heart is protected from generation of oxygen free radicals.

9. The method of claim 2 wherein said superoxide dismutase and catalase combination is administered in a cardioplegic solution.

10. The method of claim 1 wherein said mammal is a human being.

11. The method of claim 1 wherein said treatment is effected by means of bolus injection prior to heart surgery.

12. The method of claim 2 wherein said treatment is effected by means of bolus injection prior to heart surgery.

13. The method of claim 9 wherein said treatment is effected by infusion.

14. The method of claim 1 wherein said treatment is effected subsequently to heart surgery.

15. The method of claim 2 wherein said treatment is effected subsequently to heart surgery.

16. The method of claim 12 wherein said treatment is effected by means of bolus injection.

17. The method of claim 12 wherein said treatment is effected by means of infusion.

18. In a method for performing open heart surgery in a human being, the improvement which comprises administering an anti-free radical effective amount of superoxide dismutase in contact with the heart to protect the heart from structural damage during re-oxygenation.

19. The method of claim 16 wherein said superoxide dismutase is administered in solution in an amount of about 0.5 mg to about 50 mg of superoxide dismutase per kilogram body weight.

20. The method of claim 18 wherein said superoxide dismutase is administered in solution in an amount of about 1 mg to about 20 mg of superoxide dismutase per kilogram body weight.

21. The method of claim 17 wherein said superoxide dismutase is administered in combination with catalase.

22. The use of superoxide dismutase for the manufacture of a medicament for treating global ischemia.

23. A composition for use in treatment of global ischemia, comprising superoxide dismutase in a physiologically acceptable carrier vehicle.

# PARTIAL EUROPEAN SEARCH REPORT

which under Rule 45 of the European Patent Convention shall be considered, for the purposes of subsequent proceedings, as the European search report

Application number

## DOCUMENTS CONSIDERED TO BE RELEVANT

EP 88305273.0

| Category | Citation of document with indication, where appropriate, of relevant passages | Relevant to claim | CLASSIFICATION OF THE APPLICATION (Int. Cl.4) |
|---|---|---|---|
| X | WO - A1 - 87/01 387 (SYN-TEK AB) <br> * Claims 1,51,52 * | 22,23 | A 61 K 37/50 |
| X | EP - A2 - 0 213 628 (YEDA) <br> * Page 3, lines 8-21 * | 22,23 | |
| A | EP - A2 - 0 200 467 (AJINOMOTO) <br> * Page 1, lines 3-10; claims 1, 12 * | 22,23 | |

### TECHNICAL FIELDS SEARCHED (Int. Cl.4)

A 61 K 37/00
A 61 K 35/00
C 12 N 9/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 22,23
Claims searched incompletely: —
Claims not searched: 1-21
Reason for the limitation of the search: EPC Art. 52 (4) Methods for treatment of the human or animal body by surgery or therapy

| Place of search | Date of completion of the search | Examiner |
|---|---|---|
| VIENNA | 09-09-1988 | DUNGLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1.03.82